# EUROPEAN PATENT SPECIFICATION

(11) **EP 2 471 520 B1**
(45) Date of publication and mention of the grant of the patent: **20.04.2016**
(21) Application number: 11196040.7
(22) Date of filing: 29.12.2011
(51) Int. Cl.: A61K 9/20

(54) **Pharmaceutical compositions of levetiracetam**
Pharmazeutische Levetiracetam-Zusammensetzungen
Compositions pharmaceutiques de lévétiracetam

(30) Priority: 30.12.2010 TR 201011148
(43) Date of publication of application: 04.07.2012
(73) Proprietor: Sanovel Ilaç Sanayi Ve Ticaret Anonim Sirketi, 34460 Sariyer/Istanbul (TR)
(72) Inventor: Cifter, Ümit, 34398 Istanbul (TR); Türkyilmaz, Ali, 34398 Istanbul (TR); Yildirim, Ediz, 34398 Istanbul (TR); Erdem, Yelda, 34398 Istanbul (TR); Gürpinar, Hakan, 34398 Istanbul (TR)
(74) Representative: Sevinç, Erkan

(56) References cited:
- EP-A1- 2 277 511
- WO-A2-2006/123357
- WO-A2-2009/135646
- WO-A2-2011/019956
- US-A1- 2006 165 796

## Description

### Technical Field of the Invention

The present invention relates to the pharmaceutical tablet compositions of levetiracetam. Furthermore, the invention relates to the process for manufacturing the compositions and its use for the treatment of epilepsy.

### Background of the Invention

Levetiracetam is an antiepileptic drug available as immediate and extended release tablets for oral administration. The chemical name of levetiracetam, a single enantiomer, is (-)-(S)-a-ethyl-2-oxo-1-pyrrolidine acetamide, and its chemical structure is shown in the Formula I.

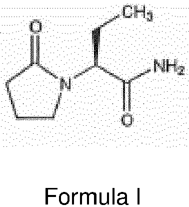

Levetiracetam is marketed under the brand name Keppra^{®} and it is administered orally in a therapeutic dose of 250 mg, 500 mg, 750 mg and 1000 mg.

Keppra tablets contain the labeled amount of levetiracetam and the inactive ingredients contain colloidal silicon dioxide, croscarmellose sodium, magnesium stearate, polyethylene glycol, polyvinyl alcohol, talc, titanium dioxide, and coloring agents.

In the case of levetiracetam high tablet dosage forms including high doses of levetiracetam are desirable, which however are often cause problems such as bad compressibility, low flowability, sticking and agglomeration of active ingredient, problems being more pronounced with high strength dosage forms (e. g. 750, 1000 mg tablets).

In order to keep the weight of the tablet at a desirable low range such as, for example, at a range of less than about 1500 mg in view of the situation that relatively larger tablets are less preferred in terms of patient compliance, the amount of excipients should be controlled. On the other hand, excipients are normally necessary to achieve satisfactory technological properties. In the case of levetiracetam, especially when used at high doses, the achievement of a good balance of technological properties, in particular appropriate flowability and compressibility, and product properties, in particular dissolution performance, is challenging.

It has been observed that, levetiracetam amount in tablet formulations is generally comprises about 85.0 % or more of the total tablet weight. Therefore, to obtain desirable tablet formulations of levetiracetam in an appropriate dosage forms, the amount of excipients should be less. On the other hand, in prior art it is seen that to overcome the problems of flowability and compressibility high amounts of binders were used.

Accordingly, a dosage form with appropriate physical characteristics avoids future problems such as chipping or breaking during packaging and transport and during tumbling in the film coating pan. If a tablet is too friable, it will chip or break during packaging and transport. In prior art it is seen that, to increase the hardness and friability of levetiracetam tablets the compression force and the proportion of binder are increased, but it has been found in each case that a sufficiently hard and non-friable tablet could not be produced in a practical way.

Another problem of the prior art is the formulating of high soluble active ingredients, such as levetiracetam, in high dosage forms. Dry granulation methods are difficultly applicable in such case because it is not easy to achieve adequate hardness thus tablets become friable and exhibit poor binding characteristics. Therefore processing of the tablet is highly influenced by the active ingredient, levetiracetam. For instance, dry granulation or direct compression methods are difficultly applicable with this compound, because it is not easy to achieve adequate hardness and the resulting tablets are friable and exhibit poor binding and compressibility characteristics and as a result of these poor properties the tablet may become unstable during the shelf-life. Therefore granulation of any sort must involve a wet granulating agent such as water but, the active, levetiracetam, is hygroscopic and for instance is very soluble in water. When too much water is sprayed, the blend can cake and become very pasty. This pasty material then proceeds to hard granules. After drying step, these granules may weight so heavy and can be very hard so that it can be difficult to compress them. Even if an alternative wet granulating agent is used, such as alcohol, and the granules do not get as drastically pasty as observed with water the drying is still an issue in that the paste remains hard and heavy unable to compress into adequate tablet weight.

Accordingly, wet granulation method applies better binding characteristics to the formulation but the granulation medium and steps has to be chosen carefully, as well as the excipients used to achieve appropriate physical characteristics of the tablet.

In prior art, there are many patents including levetiracetam formulations, such as WO 2007/012439 discloses a pharmaceutical composition which comprises levetiracetam and 2.0 to 9.0 % per weight of disintegrant, 0.0 to 3.0 % per weight of gliding agent, 0.5 to 6.0 % per weight of binder, and 0.0 to 1.0 % per weight of lubricant, with respect to the total weight of the pharmaceutical composition, as well as a process for the preparation thereof. This patent application is silent about the problems of the prior art mentioned above, and no improvement is suggested to overcome the stated problems.

Therefore, remains a need for improving the pharmacotechnical and physicochemical characteristics, regarding the storage stability and the process for manufacturing such pharmaceutical tablet compositions of levetiracetam according to the formulations currently used.

### Summary of the Invention

The main object of the present invention is therefore to provide an improved and novel pharmaceutical tablet compositions of levetiracetam for oral administration containing a high portion of levetiracetam as an active ingredient which overcomes the above described problems in prior art and have additive advantages over them.

Accordingly, another object of the present invention is to provide an improved dosage form with appropriate physical characteristics which has a sufficient hardness and friability with satisfactory disintegration time, lubrication properties and flow properties.

Another object of the present invention is to obtain stable pharmaceutical tablet compositions of levetiracetam during the shelf-life.

Another object of the present invention is to obtain better flowability and compressibility of levetiracetam during the manufacturing process without using additional binders.

A further object of the present invention is to formulating the high soluble active ingredient, levetiracetam, in high dosage forms by wet granulation method.

Moreover, it is another object of the present invention to provide a solid dosage formulation for oral administration containing levetiracetam as an active ingredient, at a high concentration, which can be prepared in dosage forms of different strength by proportionally adjusting the quantities of the excipients and the active ingredient, thereby providing excellent pharmacotechnical and physicochemical characteristics, regarding its storage stability, without affecting the dissolution profile and bioavailability of the active ingredient.

A further aspect of the present invention is to provide a method, particularly a wet granulation method, for the preparation of a stable solid dosage formulation containing a high portion of levetiracetam as an active ingredient, thereby improving the pharmacotechnical and physicochemical characteristics of the composition.

In a preferred embodiment according to the present invention said novelty is obtained with the pharmaceutical tablet composition of levetiracetam comprising,
a. crosscarmellose sodium,
b. polyvinylpyrrolidone,
c. colloidal silicon dioxide,
d. magnesium stearate,
e. coating comprising polyvinyl alcohol and xanthan gum.

According to a preferred embodiment of the present invention, the weight ratio of polyvinyl alcohol to xanthan gum is 100:1 to 1:100 (w/w).

According to a preferred embodiment of the present invention, the coating is free of hydroxypropyl methylcellulose.

According to a preferred embodiment of the present invention, said composition does not comprise additional binders which are selected from the group comprising polyethylene glycols, microcrystalline cellulose, saccharose, mannitol and sorbitol and their mixtures.

According to a preferred embodiment of the present invention, the amount of crosscarmellose sodium is from 4.0 to 10.0 %, preferably from 5.0 to 8.0%, more preferably from 5.50 to 7.0 % by weight of total composition.

According to a preferred embodiment of the present invention, the amount of polyvinylpyrrolidone is from 0.10 to 5.0 %, preferably from 1.0 to 3.0 % by weight of total composition.

According to a preferred embodiment of the present invention, the amount of colloidal silicon dioxide is from 0.01 to 3.0 %, preferably from 0.10 to 1.0 %, more preferably from 0.10 to 0.50 % by weight of total composition.

According to a preferred embodiment of the present invention, the amount of magnesium stearate is from 0.10 to 2.0 %, preferably from 0.50 to 1.0 % by weight of total composition.

According to a preferred embodiment of the present invention, the amount of levetiracetam is from 50.0 to 95.0 %, preferably from 60.0 to 95.0 % by weight of total composition.

According to a preferred embodiment of the present invention, the amount of coating is from 1.0 to 10.0 %, preferably from 1.50 to 5.0 %, more preferably from 2.0 to 3.0 % by weight of total composition.

According to a preferred embodiment of the present invention, the coating is further comprise lecithin, titanium dioxide, and coloring agents.

In a further preferred embodiment of the present invention, the pharmaceutical tablet composition of levetiracetam comprises,
a. 5.50 to 7.0 % (w/w) crosscarmellose sodium,
b. 1.0 to 3.0 % (w/w) polyvinylpyrrolidone,
c. 0.10 to 0.50 % (w/w) colloidal silicon dioxide,
d. 0.50 to 1.0 % (w/w) magnesium stearate,
e. 2.0 to 3.0 % (w/w) coating comprising polyvinyl alcohol and xanthan gum,
with respect to the total weight of the composition.

According to a preferred embodiment of the present invention, the composition is administrated orally.

According to a preferred embodiment of the present invention, the hardness of the tablet is between 100 N to 300N.

According to a preferred embodiment of the present invention, the friability of the tablet is less than 1.0 %, preferably less than 0.50 %.

Another preferred embodiment of the present invention provides a method for preparing the pharmaceutical tablet composition of levetiracetam according to present invention, which comprises the following steps;
(a) loading levetiracetam and crosscarmellose sodium into fluid bed dryer,
(b) granulating the mixture with granulation solution which comprise 44.0 : 56.0 % ethyl alcohol : water mixture with polyvinyl pyrrolidone in fluid bed dryer,
(c) grinding the granules and sieving the dry granules,
(d) mixing colloidal silicon dioxide and magnesium stearate, and blending them.
(e) compressing the blended mixture to form tablets,
(f) coating said tablets.

Further advantages and embodiments of the present invention will become apparent from the following detailed description.

### Detailed Description of the invention

According to the present invention, the improved pharmaceutical tablet composition is provided by employing suitable pharmaceutically acceptable excipients and the coating comprising polyvinyl alcohol and xanthan gum, furthermore suitable pharmacotechnical and physicochemical properties are obtained suitable for the tablet formulation with wet granulation.

The pharmacotechnical properties of pharmaceutical tablets include friability, hardness and flowability of the granules. Some of the problems that are encountered when producing tablets comprising high dose, high solubility active ingredients are friction and shock that most often cause tablets to chip or break.

Tablet friability is defined according to EP Pharmacopoeia, pharmaceutical tablets should have a friability not exceeding 1% with a Carr Index from 1 - 25, preferably from 1 -15. The friability test is closely related to the tablet hardness and is designed to evaluate the ability of the tablet to withstand abrasion during packaging and handling.

Tablet hardness constitutes another important pharmacotechnical property during tablet manufacture. Tablet hardness is associated with several tablet properties, including density and porosity. Hardness generally increases with normal storage of tablets, and depends on the shape, chemical properties, as well as pressure applied during compression. If a tablet is too hard, then it may not disintegrate in the required period of time to meet the dissolution specifications. In contrast, if a tablet is too soft, then it may not be able to withstand the handling during subsequent processing, such as coating or packaging.

Another significant important property determining the pharmacotechnical property of tablets is the flow property or fluidity. Flow property or fluidity is a characteristic required in order to produce tablets of a consistent weight and uniform strength. This means that good flowability is desirable for content uniformity and less weight variation in final tablets. In addition to that, compressibility is required to form a stable, intact compact mass when pressure is applied. Pharmaceutical formulations with acceptable flow property and compressibility characteristics for granules can be obtained by incorporating suitable excipients. Flow property of tablet granules is defined via the known term "Carr's Compressibility Index".

Furthermore, another property of significant importance during the evaluation of tablets is tablet dissolution. The dissolution rate of the drug from the primary particles of the tablet constitutes the important factor in drug absorption and for many formulations is the rate-limiting step. Therefore, the dissolution rate is an indication of the availability of the active ingredient from the tablet, especially if it is coated.

As already mentioned levetiracetam has high solubility and its tendency gets stronger when it is formulated and mixed with excipients. Therefore the selection of the excipients has an importance in this invention.

It has been surprisingly found that the objects of the present invention mentioned above are achieved by employing the pharmaceutical tablet composition of levetiracetam comprising, crosscarmellose sodium, polyvinylpyrrolidone, colloidal silicon dioxide, magnesium stearate and a coating comprising polyvinyl alcohol, preferably partially hydrolised and xanthan gum.

More specifically it has been found that, the coating comprising polyvinyl alcohol and xanthan gum improves the pharmacotechnical properties of the tablets. The emulsifiyer and adhesive enhancer properties of xanthan gum when combined with the properties of polyvinyl alcohol such as high humidty barrier property has a **synergistic effect** over the dissolution and stability of the tablet compositions of levetiracetam. Especially, when the weight ratio of polyvinyl alcohol to xanthan gum is 100:1 to 1:100 (w/w), better results are obtained. According to another embodiment of the present invention the coating is free of hydroxypropyl methylcellulose. The coating is further comprise lecithin, titanium dioxide, and coloring agents such as quinoline, FD&C dyes, Indigo carmine, sunset yellow.

Furthermore, another property of significant importance during the evaluation of tablets is to obtain better flowability and compressibility of levetiracetam during the manufacturing process. It has been provided without using additional binders. Levetiracetam amount in tablet formulations is generally comprises about 85.0 % or more of the total tablet weight.

Therefore, to obtain desirable tablet formulations of levetiracetam in an appropriate dosage forms, the amount of excipients should be less.

According to one embodiment of the present invention, the pharmaceutical tablet composition of this present invention was tested for its dissolution profile in 900 ml of water at 37ºC using a USP paddle method and levetiracetam is dissolved more than 85.0 % in 30 min., preferably it is dissolved more than 85.0% in 15 min.

Another embodiment of the present invention is the use of the wet granulation process for the preparation of pharmaceutical tablet compositions of levetiracetam, which comprises; loading levetiracetam and croscarmellose sodium to the fluid bed dryer, preparing binder solution as dissolving Polyvinylpyrrolidone K-30 in ethyl alcohol and purified water mixture (44 %: 56 %) for 10 minutes, spraying the binder solution on powder mixture in order to make granulation, drying the granule until desired loss on drying value is obtained in fluidizing bed drier with a drying temperature of 55 ± 2 °C, adding colloidal silicon dioxide to granule mixture and grinding through a 1.0 mm sized sieve at high speed., mixing the grinded granule for 15 min., adding magnesium stearate, mixing for 5 min. Compressing the blended mixture to form tablets and coating said tablets.

This invention is further defined by reference to the following examples. Although the example is not intended to limit the scope of the present invention, it should be considered in the light of the description detailed above. It will be apparent to those skilled in the art that many modifications, both to materials and methods, may be practiced without departing from the scope of the invention.

### Example 1

The pharmaceutical tablet composition of levetiracetam consisting of,
88.30 % (w/w) levetiracetam,
6.0 % (w/w) crosscarmellose sodium,
1.78 % (w/w) polyvinylpyrrolidone K-30
0.35 % colloidal silicon dioxide
0.71 % (w/w) magnesium stearate,
2.86 % (w/w) coating comprising polyvinyl alcohol-partially hydrolized, xanthan gum, lecithin, titanium dioxide and coloring agents.
with respect to the total weight of the tablet.

### Example 2

The pharmaceutical composition of this present invention was tested for its dissolution profile (Example 1 is used, 12 tablets are tested and their average value is recorded) in 900 ml of water at 37ºC using a USP paddle method rotating at 50 RPM. Results are shown in Table 1.

**Table 1**

| **Time (min.)** | **Levetiracetam (%)** |
|---|---|
| 5 | 35 |
| 10 | 63 |
| 15 | 87 |
| 20 | 93 |
| 30 | 100 |
| 45 | 101 |

### Example 3

The pharmaceutical composition of the example 1 is also tested according to their "Carr compressibility" as they are shown in Table 2. Common indices of flowability are the Carr index. The increase in bulk density of a powder is related to the cohesiveness of a powder. So measurement of the bulk density of a powder is essential to define the flow characteristics. The Carr index gives us the guidance for powder flowability. A lower Carr index of excipients is more desirable for acceptable powder flow. Carr Index Classification and Powder Flowability is shown below (according to European Pharmacopeia 7.0);

| Carr Index (compressibility index) | (%) Flow character |
|---|---|
| 1 - 10 | Excellent |
| 11 - 15 | Good |
| 16 - 20 | Fair |
| 21 - 25 | Passable |
| 26 - 31 | Poor |
| 32 - 37 | Very Poor |
| > 38 | very, very poor |

**Table 2**

| **Example** | **1** |
|---|---|
| **Carr Compressibilty** (%) | 9.5 |

## Claims

1. A pharmaceutical tablet composition of levetiracetam comprising,
a. 4.0 to 10.0 % (w/w) crosscarmellose sodium,
b. 0.10 to 5.0 % (w/w) polyvinylpyrrolidone,
c. 0.01 to 3.0 % (w/w) colloidal silicon dioxide,
d. 0.10 to 2.0 % (w/w) magnesium stearate,
e. 1.0 to 10.0 % (w/w) coating comprising polyvinyl alcohol and xanthan gum.

2. The pharmaceutical tablet composition of levetiracetam according to claim 1, wherein the weight ratio of polyvinyl alcohol to xanthan gum is 100:1 to 1:100 (w/w).

3. The pharmaceutical tablet composition of levetiracetam according to claims 1 and 2, wherein the coating is free of hydroxypropyl methylcellulose.

4. The pharmaceutical tablet composition of levetiracetam according to claims 1 to 3, wherein said composition does not comprise additional binders which are selected from the group comprising polyethylene glycols, microcrystalline cellulose, saccharose, mannitol and sorbitol and their mixtures.

5. The pharmaceutical tablet composition of levetiracetam according to claims 1 to 4, wherein the amount of crosscarmellose sodium is from 5.0 to 8.0%, preferably from 5.50 to 7.0 % by weight of total composition.

6. The pharmaceutical tablet composition of levetiracetam according to claims 1 to 4, wherein the amount of polyvinylpyrrolidone is from 1.0 to 3.0 % by weight of total composition.

7. The pharmaceutical tablet composition of levetiracetam according to claims 1 to 4, wherein the amount of colloidal silicon dioxide is from 0.10 to 1.0 %, more preferably from 0.10 to 0.50 % by weight of total composition.

8. The pharmaceutical tablet composition of levetiracetam according to claims 1 to 4, wherein the amount of magnesium stearate is from 0.50 to 1.0 % by weight of total composition.

9. The pharmaceutical tablet composition of levetiracetam according to claims 1 to 4, wherein the amount of levetiracetam is from 50.0 to 95.0 %, preferably from 60.0 to 95.0 % by weight of total composition.

10. The pharmaceutical tablet composition of levetiracetam according to any preceding claims, wherein the amount of coating is from from 1.50 to 5.0 %, more preferably from 2.0 to 3.0 % by weight of total composition.

11. The pharmaceutical tablet composition of levetiracetam according to any preceding claims, wherein the coating is further comprise lecithin, titanium dioxide, and coloring agents.

12. The pharmaceutical tablet composition of levetiracetam according to any preceding claims comprising,
a. 5.50 to 7.0 % (w/w) crosscarmellose sodium,
b. 1.0 to 3.0 % (w/w) polyvinylpyrrolidone,
c. 0.10 to 0.50 % (w/w) colloidal silicon dioxide,
d. 0.50 to 1.0 % (w/w) magnesium stearate,
e. 2.0 to 3.0 % (w/w) coating comprising polyvinyl alcohol and xanthan gum,
with respect to the total weight of the composition.

13. The pharmaceutical tablet composition of levetiracetam according to any preceeding claims, wherein the composition is administrated orally.

14. The pharmaceutical tablet composition of levetiracetam according to any preceeding claims, wherein the hardness of the tablet is between 100 N to 300N.

15. The pharmaceutical tablet composition of levetiracetam according to any preceeding claims, wherein the friability of the tablet is less than 1.0 %, preferably is less than 0.50 %.

16. A wet granulation process for preparing the pharmaceutical tablet composition of levetiracetam of any preceeding claims which comprises the following steps;
(a) loading levetiracetam and crosscarmellose sodium into fluid bed dryer,
(b) granulating the mixture with granulation solution which comprise 44.0:56.0 % ethyl alcohol:water mixture with polyvinyl pyrrolidone in fluid bed dryer,
(c) grinding the granules and sieving the dry granules,
(d) mixing colloidal silicon dioxide and magnesium stearate, and blending them.
(e) compressing the blended mixture to form tablets,
(f) coating said tablets.

## Patentansprüche

1. Pharmazeutische Tablettenzusammensetzung von Levetiracetam, umfassend
a. 4,0 bis 10,0 % (w/w) Crosscarmellose-Natrium,
b. 0,10 bis 5,0 % (w/w) Polyvinylpyrrolidon,
c. 0,01 bis 3,0 % (w/w) kolloidales Siliziumdioxid,
d. 0,10 bis 2,0 % (w/w) Magnesiumstearat,
e. 1,0 bis 10,0 % (w/w) Überzug, der Polyvinylalkohol und Xanthangummi umfasst.

2. Pharmazeutische Tablettenzusammensetzung von Levetiracetam nach Anspruch 1, wobei das Gewichtsverhältnis von Polyvinylalkohol zu Xanthangummi 100:1 bis 1:100 (w/w) beträgt.

3. Pharmazeutische Tablettenzusammensetzung von Levetiracetam nach den Ansprüchen 1 und 2, wobei der Überzug frei von Hydroxypropylmethylcellulose ist.

4. Pharmazeutische Tablettenzusammensetzung von Levetiracetam nach den Ansprüchen 1 bis 3, wobei die Zusammensetzung keine zusätzlichen Bindemittel umfasst, die aus der Polyethylenglykole, mikrokristalline Cellulose, Saccharose, Mannit und Sorbit und deren Gemische umfassenden Gruppe ausgewählt sind.

5. Pharmazeutische Tablettenzusammensetzung von Levetiracetam nach den Ansprüchen 1 bis 4, wobei die Menge an Crosscarmellose-Natrium von 5,0 bis 8,0 Gew.-%, vorzugsweise von 5,50 bis 7,0 Gew.-%, bezogen auf das Gewicht der Gesamtzusammensetzung, beträgt.

6. Pharmazeutische Tablettenzusammensetzung von Levetiracetam nach den Ansprüchen 1 bis 4, wobei die Menge an Polyvinylpyrrolidon von 1,0 bis 3,0 Gew.-%, bezogen auf das Gewicht der Gesamtzusammensetzung, beträgt.

7. Pharmazeutische Tablettenzusammensetzung von Levetiracetam nach den Ansprüchen 1 bis 4, wobei die Menge an kolloidalem Siliziumdioxid von 0, 10 bis 1,0 Gew.-%, stärker bevorzugt von 0,10 bis 0,50 Gew.-%, bezogen auf das Gewicht der Gesamtzusammensetzung, beträgt.

8. Pharmazeutische Tablettenzusammensetzung von Levetiracetam nach den Ansprüchen 1 bis 4, wobei die Menge an Magnesiumstearat von 0,50 bis 1,0 Gew.-%, bezogen auf das Gewicht der Gesamtzusammensetzung, beträgt.

9. Pharmazeutische Tablettenzusammensetzung von Levetiracetam nach den Ansprüchen 1 bis 4, wobei die Menge an Levetiracetam von 50,0 bis 95,0 Gew.-%, vorzugsweise von 60,0 bis 95,0 Gew.-%, bezogen auf das Gewicht der Gesamtzusammensetzung, beträgt.

10. Pharmazeutische Tablettenzusammensetzung von Levetiracetam nach einem der vorhergehenden Ansprüche, wobei die Menge an Überzug von 1,50 bis 5,0 Gew.-%, stärker bevorzugt von 2,0 bis 3,0 Gew.-%, bezogen auf das Gewicht der Gesamtzusammensetzung, beträgt.

11. Pharmazeutische Tablettenzusammensetzung von Levetiracetam nach einem der vorhergehenden Ansprüche, wobei der Überzug weiterhin Lecithin, Titandioxid und Färbemittel umfasst.

12. Pharmazeutische Tablettenzusammensetzung von Levetiracetam nach einem der vorhergehenden Ansprüche, umfassend
a. 5,50 bis 7,0 % (w/w) Crosscarmellose-Natrium,
b. 1,0 bis 3,0 % (w/w) Polyvinylpyrrolidon,
c. 0,10 bis 0,50 % (w/w) kolloidales Siliziumdioxid,
d. 0,50 bis 1,0 % (w/w) Magnesiumstearat,
e. 2,0 bis 3,0 % (w/w) Überzug, der Polyvinylalkohol und Xanthangummi umfasst, bezogen auf das Gesamtgewicht der Zusammensetzung.

13. Pharmazeutische Tablettenzusammensetzung von Levetiracetam nach einem der vorhergehenden Ansprüche, wobei die Zusammensetzung oral verabreicht wird.

14. Pharmazeutische Tablettenzusammensetzung von Levetiracetam nach einem der vorhergehenden Ansprüche, wobei die Härte der Tablette zwischen 100 N bis 300 N beträgt.

15. Pharmazeutische Tablettenzusammensetzung von Levetiracetam nach einem der vorhergehenden Ansprüche, wobei die Zerreibbarkeit der Tablette kleiner als 1,0 % ist, vorzugsweise kleiner als 0,50 % ist.

16. Feuchtgranulationsverfahren zur Herstellung der pharmazeutischen Tablettenzusammensetzung von Levetiracetam nach einem der vorhergehenden Ansprüche, das die folgenden Schritte umfasst:
(a) Beschicken eines Wirbelschichttrockners mit Levetiracetam und Crosscarmellose-Natrium,
(b) Granulieren des Gemischs mit Granulationslösung, die ein 44,0:56,0 % Ethylalkohol:Wasser-Gemisch umfasst, mit Polyvinylpyrrolidon im Wirbelschichttrockner,
(c) Mahlen des Granulats und Sieben des trockenen Granulats,
(d) Mischen von kolloidalem Siliziumdioxid und Magnesiumstearat und deren Vermengung,
(e) Pressen des vermengten Gemischs zur Herstellung von Tabletten,
(f) Überziehen der Tabletten.

## Revendications

1. Composition pharmaceutique de comprimé de lévétiracétam comprenant,
a. 4,0 à 10,0% (p / p) de sodium croscarmellose,
b. 0,10 à 5,0% (p / p) de polyvinylpyrrolidone,
c. 0,01 à 3,0% (p / p) de dioxyde de silicium colloïdal,
d. 0,10 à 2,0% (p / p) de stéarate de magnésium,
e. 1,0 à 10,0% (p / p) d'enrobage comprenant de l'alcool polyvinylique et de la gomme de xanthane.

2. Composition pharmaceutique de comprimé de lévétiracétam selon la revendication 1, dans lequel le rapport en poids de l'alcool polyvinylique sur la gomme de xanthane est de 100:1 à 1:100 (p / p).

3. Composition pharmaceutique de comprimé de lévétiracétam selon l'une des revendications 1 et 2, dans lequel l'enrobage est exempt d'hydroxypropyl-méthylcellulose.

4. Composition pharmaceutique de comprimé de lévétiracétam selon l'une des revendications 1 à 3, dans lequel ladite composition ne comprend pas de liants supplémentaires qui sont choisis dans le groupe comprenant les polyéthylène glycols, la cellulose microcristalline, le saccharose, le mannitol et le sorbitol et leurs mélanges.

5. Composition pharmaceutique de comprimé de lévétiracétam selon les revendications 1 à 4, dans lequel la quantité de sodium croscarmellose est de 5,0 à 8,0%, de préférence de 5,50 à 7,0% en poids de la composition totale.

6. Composition pharmaceutique de comprimé de lévétiracétam selon les revendications 1 à 4, dans lequel la quantité de polyvinylpyrrolidone est de 1,0 à 3,0% en poids de la composition totale.

7. Composition pharmaceutique de comprimé de lévétiracétam selon les revendications 1 à 4, dans laquelle la quantité de dioxyde de silicium colloïdal est de 0,10 à 1,0%, plus préférablement de 0,10 à 0,50% en poids de la composition totale.

8. Composition pharmaceutique de comprimé de lévétiracétam selon les revendications 1 à 4, dans laquelle la quantité de stéarate de magnésium est de 0,50 à 1,0% en poids de la composition totale.

9. Composition pharmaceutique pour comprimés de lévétiracétam selon les revendications 1 à 4, dans laquelle la quantité du lévétiracétam est de 50,0 à 95,0%, de préférence de 60,0 à 95,0% en poids de la composition totale.

10. Composition pharmaceutique de comprimé de lévétiracétam selon l'une quelconque des revendications précédentes, dans laquelle la quantité d'enrobage est de 1,50 à 5,0%, plus préférablement de 2,0 à 3,0% en poids de la composition totale.

11. Composition pharmaceutique de comprimé de lévétiracétam selon l'une quelconque des revendications précédentes, dans laquelle l'enrobage comprend en outre de la lécithine, du dioxyde de titane, et des agents colorants.

12. Composition pharmaceutique de comprimé de lévétiracétam selon l'une quelconque des revendications précédentes, comprenant,
a. 5,50 à 7,0% (p / p) de sodium croscarmellose,
b. 1 à 3% (p / p) de polyvinylpyrrolidone,
c. 0,10 à 0,50% (p / p) de dioxyde de silicium colloïdal,
d. 0,50 à 1% (p / p) de stéarate de magnésium,
e. 2,0 à 3,0% (p / p) d'enrobage comprenant de l'alcool polyvinylique et de la gomme de xanthane,
par rapport au poids total de la composition.

13. Composition pharmaceutique de comprimé de lévétiracétam selon une quelconque des revendications précédentes, dans laquelle la composition est administrée par voie orale.

14. Composition pharmaceutique de comprimé de lévétiracétam selon une quelconque des revendications précédentes, dans laquelle la dureté du comprimé est comprise entre 100 N et 300 N.

15. Composition pharmaceutique de comprimé de lévétiracétam selon l'une quelconque des revendications précédentes, dans laquelle la friabilité du comprimé est inférieure à 1,0%, de préférence inférieure à 0,50%.

16. Procédé de granulation par voie humide pour la préparation de la composition pharmaceutique de comprimé de lévétiracétam selon l'une quelconque des revendications précédentes, qui comprend les étapes suivantes consistant à ;
(a) charger le lévétiracétam et le sodium de croscarmellose dans un séchoir à lit fluide,
(b) granuler le mélange avec une solution de granulation qui comprend 44,0:56,0% d'alcool éthylique:de mélange d'eau avec de la polyvinylpyrrolidone dans un séchoir à lit fluide,
(c) broyer les granules et les granules secs sievingthe,
(d) mélanger du dioxyde de silicium colloïdal et du stéarate de magnésium, et les agiter,
(e) comprimer le mélange agiter pour former des comprimés,
(f) enrober lesdits comprimés.
